# EUROPÄISCHE PATENTANMELDUNG

(11) **EP 2 016 923 A1**
(43) Veröffentlichungstag der Anmeldung: **21.01.2009**
(21) Anmeldenummer: 07013840.9
(22) Anmeldetag: 14.07.2007
(51) Int. Cl.: A61F 2/30, A61F 2/36

(54) **Gelenk-Prothese**

(71) Anmelder: Walther, Gerd Axel, Dr., 71034 Böblingen (DE)
(72) Erfinder: Walther, Gerd Axel, Dr., 71034 Böblingen (DE)
(74) Vertreter: Vetter, Hans

(57) **Zusammenfassung**

Es wird eine Gelenk-Prothese mit einer in das Knochengewebe einsetzbaren ersten Prothesenkomponente (10) vorgeschlagen, auf der ein z.B. als Kugelkopf ausgebildeter Gelenkkopf aus einem Keramikmaterial aufgesetzt ist, welcher zusammen mit einer zweiten, eine Gelenkpfanne aufweisenden Prothesenkomponente ein Prothesen-Gelenk bildet. Der Gelenkkopf auf der ersten Prothesenkomponente (10) wird mittels einer als Steck-Drehverbindung ausgebildeten Renkverbindung fixiert.

## Beschreibung

Die Erfindung betrifft eine Gelenk-Prothese mit einer in das Knochengewebe einsetzbaren ersten Prothesenkomponente, auf der ein Gelenkkopf aus einem Keramikmaterial aufgesetzt ist, welcher zusammen mit einer zweiten, eine Gelenkpfanne aufweisenden Prothesenkomponente ein Prothesen-Gelenk bildet.

Bei einer derartigen, aus der DE 19904437 A1 bekannten Gelenk-Prothese mit Kugelgelenk wird ein kreiskegelstumpfförmiger Haltevorsprung an der ersten Prothesenkomponente in eine entsprechende Aufnahme des Kugelkopfes eingesetzt und lediglich durch konische Verklemmung fixiert. Hierzu ist an der Klemmfläche zwischen dem Haltevorsprung und der Aufnahme ein Kopplungselement angeordnet, das als Fäden aufweisender Wickelkörper oder Sinterkörper oder Schwammkörper ausgebildet ist. Bei diesem bekannten reinen Klemmsitz besteht allerdings die Gefahr, dass sich der Kugelkopf infolge mechanischer Einflüsse, Materialermüdung oder Alterung löst, was eine nicht unerhebliche Operation zur Folge hat. Eine ähnliche Anordnung ist aus der DE 19813074 A1 bekannt, bei der wiederum die Klemmfläche mit einem die Haftung verbessernden Überzug versehen ist, nicht zuletzt auch deshalb, da sich auf der einen Seite der Klemmfläche ein Keramikmaterial und auf der anderen Seite ein Metall, beispielsweise Titan, befindet. Die unterschiedlichen Eigenschaften dieser Materialien erhöhen die Gefahr des Lösens der Klemmverbindung.

Die in das Knochengewebe einsetzbare erste Prothesenkomponente besteht gewöhnlich aus einem Metall, wie Titan oder einer Titanlegierung, das sich leichter bearbeiten lässt und beim Einschlagen höhere Belastungen aushält. Dagegen besteht der z.B. als Kugelkopf ausgebildete Gelenkkopf gewöhnlich aus einem Keramikmaterial, das einen wesentlich geringeren Abrieb infolge der größeren Härte aufweist und somit eine längere Lebensdauer gewährleistet.

Eine Aufgabe der vorliegenden Erfindung besteht darin, eine Verbindung zwischen Gelenkkopf und der ersten Prothesenkomponente aufzuzeigen, die auch bei unterschiedlichen Materialien über sehr lange Zeit eine sichere, unlösbare Verbindung gewährleistet.

Diese Aufgabe wird erfindungsgemäß durch eine Gelenk-Prothese mit den Merkmalen des Anspruchs 1 gelöst.

Die erfindungsgemäße Verankerung des Gelenkkopfs an der ersten Prothesenkomponente durch eine als Steck-Drehverbindung ausgebildete Renkverbindung stellt eine sichere, formschlüssige Verbindung dar und kann durch einen einfachen Steck-Drehvorgang um einen wahlweise kleinen Winkel hergestellt werden. Insbesondere bei unterschiedlichen miteinander zu verbindenden Materialien wird eine sichere und haltbare Verbindung erreicht, die sich auch bei Materialveränderungen und Alterung nicht lösen kann. Zur Herstellung der Verbindung ist ein relativ geringer Kraftaufwand erforderlich, was im Laufe einer Gelenkoperation durchaus von Vorteil ist. Weiterhin besteht die einfache Möglichkeit, den Gelenkkopf wieder von der ersten Prothesenkomponente durch Drehen in der entgegengesetzten Richtung zu lösen, falls sich aus unterschiedlichen Gründen ein anders dimensionierter Gelenkkopf während der Operation als günstiger erweisen sollte.

Durch die in den Unteransprüchen aufgeführten Maßnahmen sind vorteilhafte Weiterbildungen und Verbesserungen der im Anspruch 1 angegebenen Gelenk-Prothese möglich.

In einer vorteilhaften konstruktiven Ausgestaltung der Erfindung ist die erste Prothesenkomponente oder der insbesondere als Kugelkopf ausgebildete Gelenkkopf mit einem in eine entsprechend geformte Aufnahme des jeweils anderen Teils einsteckbaren Haltevorsprung versehen, der eine radial vorstehende Verriegelungselementanordnung besitzt, die durch eine Komplementärkontur in der Aufnahme hindurch in diese einführbar und durch Drehung hinter dieser Komplementärkontur verriegelbar ist. Dieser Haltevorsprung ist bevorzugt an der ersten Prothesenkomponente angeordnet, sodass der Gelenkkopf entsprechend die Aufnahme besitzt.

Die Verriegelungselementanordnung besitzt wenigstens ein zahnartiges Verriegelungselement, vorzugsweise eine symmetrisch verteilte Anzahl von Verriegelungselementen, wobei dann die Komplementärkontur eine oder mehrere entsprechende Durchsteckausnehmungen besitzt. Dies gewährleistet eine besonders schnelle und einfache Verriegelung des Gelenkkopfes an der ersten Prothesenkomponente bei relativ geringem Herstellungsaufwand.

Die hinter der Komplementärkontur verriegelte oder verriegelbare Verriegelungselementanordnung ist in vorteilhafter Weise zur Verklemmung des Haltevorsprungs in der Aufnahme ausgebildet, das heißt, bei der Drehung verklemmen sich die Verriegelungselemente hinter der Komplementärkontur. Dies führt zu einer Verdrehsicherheit. Eine solche Verdrehsicherheit kann auch als formschlüssige Verdrehsicherung dadurch realisiert werden, dass wenigstens ein zapfenförmiges oder gekrümmtes, plattenförmiges Füll- und Arretierelement zum Einstecken in die mit der Verriegelungselementanordnung verriegelte Komplementärkontur ausgebildet ist, wobei dieses Füll- und Arretierelement mit dem Haltevorsprung verschraubt, verklemmt oder verklebt ist. Zweckmäßigerweise ist eine zum Ausfüllen aller nach der Verriegelung verbleibenden Durchsteckausnehmungen der Komplementärkontur erforderliche Zahl von Füll- und Verriegelungselementen vorgesehen. Neben der Verriegelung dient dieses wenigstens eine Füll- und Arretierelement auch zum Verschließen aller verbleibenden Öffnungen.

Die Füll- und Verriegelungselemente können auch wenigstens teilweise miteinander verbunden sein, insbesondere über ein Ringelement oder Teilringelemente. Damit ist insbesondere bei einer größeren Zahl von Durchsteckausnehmungen eine schnellere Montage möglich und eine einfachere Fixierung am Haltevorsprung.

Wenigstens der freie Endbereich des Haltevorsprungs und der entsprechende Anlagebereich in der Ausnehmung besitzen eine kreiskegelstumpfförmige Gestalt, was die Halteverklemmung fördert und verbessert.

Die erste Prothesenkomponente besteht zweckmäßigerweise aus Metall, insbesondere Titan oder einer Titanlegierung, um eine möglichst hohe Bruchfestigkeit und leichte Bearbeitbarkeit zu erreichen. Prinzipiell kann diese erste Prothesenkomponente jedoch aus einem Keramikmaterial bestehen, insbesondere auf der Basis von Zirkonoxid oder einer Zirkonoxid-/Aluminiumoxidmischung. Der Gelenkkopf selbst sollte zweckmäßigerweise aus einem solchen Keramikmaterial bestehen, um eine möglichst hohe Abriebfestigkeit zu erreichen.

Ein Ausführungsbeispiel der Erfindung ist in der Zeichnung dargestellt und in der nachfolgenden Beschreibung näher erläutert. Es zeigen:
- Figur 1: eine Seitenansicht eines auf eine erste Prothe- senkomponente aufsetzbaren als Gelenkkopf ausgebil- deten Gelenkkopfs, der eine Aufnahme für einen Hal- tevorsprung der ersten Prothesenkomponente besitzt,
- Figur 2: eine Querschnittdarstellung des Kugelkopfes gemäß der Schnittlinie A-A,
- Figur 3: eine Seitenansicht der mit einem Haltevorsprung versehenen ersten Prothesenkomponente,
- Figur 4: eine Draufsicht auf die freie Stirnfläche des Haltevorsprungs der ersten Prothesenkomponente und
- Figur 5: ein Füll- und Arretierelement zum Verschließen und Verriegeln der Durchsteckausnehmungen im Kugelkopf.

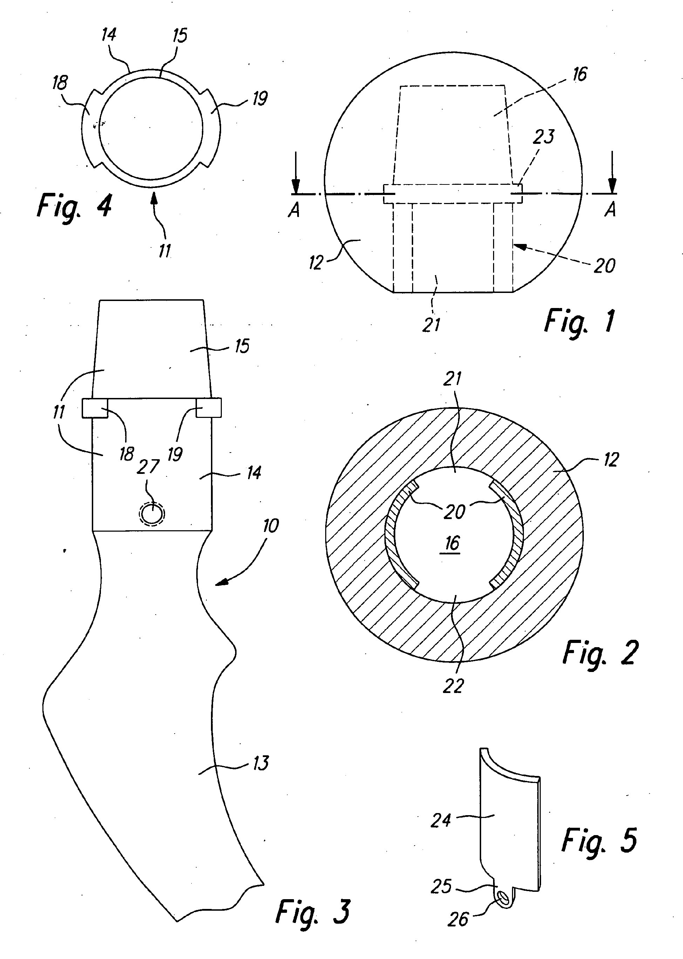

In den Figuren 1 bis 5 ist als Ausführungsbeispiel eine erste, in das Knochengewebe einsetzbare Prothesenkomponente 10 dargestellt, die einen Haltevorsprung 11 aufweist, auf den ein Kugelkopf 12 aufsteckbar und verankerbar ist. Die erste Prothesenkomponente 10 besteht aus einem in das Knochengewebe in an sich bekannter Weise einsetzbaren Knochenverankerungsbereich 13, der nur teilweise dargestellt ist und an dem am aus dem Knochen herausragenden freien Endbereich ein Haltevorsprung 11 einstückig angeformt ist. Die mit dem Kugelkopf 12 versehene erste Prothesenkomponente 10 bildet zusammen mit einer zweiten, nicht dargestellten Prothesenkomponente, die eine Gelenkpfanne zur Aufnahme des Kugelkopfes 12 aufweist und die ebenfalls in einem Knochengewebe verankerbar ist, eine mit einem Kugelgelenk versehene Gelenk-Prothese. Es handelt sich dabei beispielsweise um eine Prothese für ein Hüftgelenk oder Schultergelenk.

Die Erfindung ist selbstverständlich auch für andere Gelenke, wie Kniegelenke, Fußgelenke, Handgelenke, Kiefergelenke oder dergleichen anwendbar. Anstelle eines Kugelkopfes 12 zur Bildung eines Kugelgelenks tritt dann der für die jeweilige Anwendung erforderliche Gelenkkopf, beim Kniegelenk beispielsweise ein Walzenkopf zur Bildung eines Schwenkgelenks.

Der Haltevorsprung 11 besitzt einen zylindrischen Bereich 14, der zum freien Ende hin in einen kreiskegelstumpfförmigen Bereich 15 übergeht und sich entsprechend zum Ende hin verjüngt. Der Kugelkopf 12 besitzt ein entsprechend geformtes Sackloch 16 als Aufnahme für den Haltevorsprung 11. Zur Fixierung des Haltevorsprungs 11 im Sackloch 16 des Kugelkopfes 12 dient eine als Steck-Drehverbindung ausgebildete Renkverbindung 17. Hierzu besitzt der Haltevorsprung 11 am Übergangsbereich zwischen dem zylindrischen Bereich 14 und dem kreiskegelstumpfförmigen Bereich 15 zwei radial abstehende segment- oder zahnartige Verriegelungselemente 18, 19, während im Sackloch 16 eine entsprechende Komplementärkontur 20 angeordnet ist, die entsprechende nutartige Durchsteckausnehmungen 21, 22 zum Durchstecken der Verriegelungselemente 18, 19 besitzt. Zur gegenseitigen Verriegelung wird der Haltevorsprung 11 so in das Sackloch 16 des Kugelkopfes 12 eingeführt, dass die Verriegelungselemente 18, 19 durch die Durchsteckausnehmungen 21, 22 der Komplementärkontur 20 hindurchgeführt werden. Anschließend wird der Kugelkopf 12 um einen Winkel gedreht, der im Wesentlichen der Winkelbreite eines Verriegelungselements 18 beziehungsweise 19 entspricht. Hierdurch gelangen die Verriegelungselemente 18, 19 hinter die Komplementärkontur 20. Der kreiskegelstumpfförmige Bereich 15 in Verbindung mit dem entsprechend geformten Bereich des Sackloches 16 und in Verbindung mit den Verriegelungselementen 18, 19 und der Komplementärkontur 20 sind so geformt und ausgebildet, dass sich bei dieser Drehung des Kugelkopfes 12 die Verriegelungselemente 18, 19 hinter der Komplementärkontur 20 verklemmen, das heißt, der Haltevorsprung 11 der ersten Prothesenkomponente 10 wird dabei in das Sackloch 16 eingedrückt unter Bildung einer konischen Verklemmung.

Beim dargestellten Ausführungsbeispiel sind zwei Verriegelungselemente 18, 19 und zwei entsprechende Durchsteckausnehmungen 21, 22 gleichmäßig über den Umfang des Haltevorsprungs 11 verteilt, also gegenüberliegend angeordnet. Diese Zahl kann natürlich variieren. Im einfachsten Fall genügt ein einziges Verriegelungselement und eine einzige Durchsteckausnehmung. Die Zahl der gleichmäßig über den Umfang verteilten Verriegelungselemente kann jedoch auch größer sein. Weiterhin können die Verriegelungselemente 18, 19, die Durchsteckausnehmungen 21, 22 und die Ringnut 23 auch eine von der rechteckigen beziehungsweise quaderförmigen Gestalt oder Querschnittsgestalt abweichende Geometrie besitzen und zum Beispiel halbrund, dreieckig, elliptisch oder dergleichen ausgebildet sein.

Nach dem Verriegeln des Kugelkopfes 12 am Haltevorsprung 11 durch den beschriebenen Steck-Drehvorgang sind die Durchsteckausnehmungen 21, 22 wieder offen. Zum Verschließen derselben werden nun zwei in Figur 5 dargestellte Füll- und Arretierelemente 24 eingesteckt. Neben dem Ausfüllen der Durchsteckausnehmungen 21, 22 verriegeln sie die Position des Kugelkopfes 12 am Haltevorsprung 11 und verhindern eine Drehung und damit ein Ablösen des Kugelkopfes 12 von der ersten Prothesenkomponente 10. Diese Füll- und Arretierelemente 24 besitzen eine Gestalt, die der der Durchsteckausnehmungen 21, 22 entspricht, also im Ausführungsbeispiel die Gestalt einer gekrümmten Platte. Ein unterer Fortsatz 25 besitzt ein Durchgangsloch 26, wobei diese Durchgangslöcher 26 im eingesteckten Zustand der Füll- und Arretierelemente 24 mit entsprechenden Gewindebohrungen 27 im Haltevorsprung 11 fluchten. Durch Halteschrauben können dadurch die Füll- und Arretierelemente 24 am Haltevorsprung 11 fixiert werden. Alternative Arten der Fixierung sind selbstverständlich ebenfalls möglich, so können die Füll- und Arretierelemente 24 auch am Haltevorsprung 11 verklemmt, verklebt oder verrastet werden.

Bei der Operation zur Anbringung einer solchen Gelenk-Prothese wird zunächst nach entsprechender vorbereitung die erste Prothesenkomponente 10 im Knochengewebe, beispielsweise eines Oberschenkelknochens, verankert. Danach wird in der beschriebenen Weise der Kugelkopf 12 aufgesetzt. Die andere, nicht dargestellte zweite Prothesenkomponente wird entsprechend am Knochengewebe beispielsweise des Beckens in entsprechender Weise verankert, wobei dann der Kugelkopf 12 in die Gelenkpfanne dieser zweiten Prothesenkomponente eingesetzt werden kann. Es ist auch denkbar, dass der Kugelkopf 12 bereits in der Gelenkpfanne der zweiten Prothesenkomponente eingesetzt ist und im eingesetzten Zustand mit dem Haltevorsprung 11 der ersten Prothesenkomponente 10 über die Renkverbindung 17 fixiert wird.

Die erste Prothesenkomponente 10 und auch die nicht dargestellte zweite Prothesenkomponente bestehen üblicherweise aus Metall, insbesondere aus Titan oder einer Titanlegierung, wobei prinzipiell als Material auch ein Keramikmaterial verwendet werden kann, insbesondere ein Keramikmaterial auf der Basis von Zirkonoxid oder einer Zirkonoxid-/Aluminiumoxidmischung. Der Kugelkopf 12 besteht aus einem solchen Keramikmaterial, das gegenüber einem Metall eine wesentliche größere Abriebfestigkeit und damit längere Lebensdauer gewährleistet.

## Patentansprüche

1. Gelenk-Prothese mit einer in das Knochengewebe einsetzbaren ersten Prothesenkomponente (10), auf der ein Gelenkkopf (12) aus einem Keramikmaterial aufgesetzt ist, welcher zusammen mit einer zweiten, eine Gelenkpfanne aufweisenden Prothesenkomponente ein Prothesen-Gelenk bildet, **dadurch gekennzeichnet, dass** der Gelenkkopf (12) auf der ersten Prothesenkomponente (10) mittels einer als Steck-Drehverbindung ausgebildeten Renkverbindung fixiert ist.

2. Gelenk-Prothese nach Anspruch 1, **dadurch gekennzeichnet, dass** der Gelenkkopf (12) als Kugelkopf ausgebildet ist.

3. Gelenk-Prothese nach Anspruch 1 oder 2, **dadurch gekennzeichnet, dass** die erste Prothesenkomponente (10) oder der Gelenkkopf (11) mit einem in eine entsprechend geformte Aufnahme (16) des jeweils anderen Teils einsteckbaren Haltevorsprung (11) versehen ist, der eine radial vorstehende Verriegelungselementanordnung (18, 19) besitzt, die durch eine Komplementärkontur (20) in der Aufnahme (16) hindurch in diese einführbar und durch Drehung hinter dieser Komplementärkontur (20) verriegelbar ist.

4. Gelenk-Prothese nach Anspruch 3, **dadurch gekennzeichnet, dass** die Verriegelungselementanordnung (18, 19) wenigstens ein zahnartiges Verriegelungselement, vorzugsweise eine symmetrisch verteilte Anzahl von Verriegelungselementen, und die Komplementärkontur (20) eine oder mehrere entsprechende Durchsteckausnehmungen (21, 22) besitzt.

5. Gelenk-Prothese nach Anspruch 3 oder 4, **dadurch gekennzeichnet, dass** die hinter der Komplementärkontur (20) verriegelte oder verriegelbare Verriegelungselementanordnung (18, 19) zur Verklemmung des Haltevorsprungs (11) in der Aufnahme (16) ausgebildet ist.

6. Gelenk-Prothese nach einem der Ansprüche 3 bis 5, **dadurch gekennzeichnet, dass** wenigstens der freie Endbereich (15) des Haltevorsprungs (11) und der entsprechende Anlagebereich in der Ausnehmung (16) eine kreiskegelstumpfförmige Gestalt besitzen.

7. Gelenk-Prothese nach einem der Ansprüche 3 bis 6, **dadurch gekennzeichnet, dass** wenigstens ein zapfenförmiges oder gekrümmtes, plattenförmiges Füll- und Arretierelement (24) zum Einstecken in die mit der Verriegelungselementanordnung (18, 19) verriegelte Komplementärkontur (20) ausgebildet ist, wobei dieses wenigstens eine Füll- und Arretierelement (24) mit dem Haltevorsprung (11) verschraubt, verklemmt oder verklebt ist.

8. Gelenk-Prothese nach Anspruch 7, **dadurch gekennzeichnet, dass** eine zum Ausfüllen aller nach der Verriegelung verbleibenden Durchsteckausnehmungen (21, 22) der Komplementärkontur (20) erforderliche Zahl von Füll- und Verriegelungselementen vorgesehen ist.

9. Gelenk-Prothese nach Anspruch 7, **dadurch gekennzeichnet, dass** die Füll- und Verriegelungselemente (18, 19) wenigstens teilweise miteinander verbunden sind, insbesondere über ein Ringelement oder über Teilringelemente.

10. Gelenk-Prothese nach einem der Ansprüche 3 bis 9, **dadurch gekennzeichnet, dass** der Haltevorsprung (11) an der ersten Prothesenkomponente (10) angeordnet ist.

11. Gelenk-Prothese nach einem der vorhergehenden Ansprüche, **dadurch gekennzeichnet, dass** die erste Prothesenkomponente (10) aus Metall, insbesondere Titan oder einer Titanlegierung, oder aus einem Keramikmaterial, insbesondere auf der Basis von Zirkonoxid oder einer Zirkonoxid-/Aluminiumoxidmischung, besteht.
